# EUROPEAN PATENT SPECIFICATION

(11) **EP 2 964 325 B1**
(45) Date of publication and mention of the grant of the patent: **01.02.2017**
(21) Application number: 13712160.4
(22) Date of filing: 05.03.2013
(51) Int. Cl.: A61N 5/06

(54) **SYSTEM FOR COCHLEA STIMULATION**
SYSTEM ZUR COCHLEA-STIMULATION
SYSTÈME DE STIMULATION DE COCHLÉE

(43) Date of publication of application: 13.01.2016
(73) Proprietor: Advanced Bionics AG, 8712 Stäfa (CH)
(72) Inventor: BOYLE, Patrick, Beckenham Kent BR3 1LZ (GB)
(74) Representative: Schwan Schorer & Partner mbB
(86) International application number: PCT/EP2013/054392
(87) International publication number: WO 2014/135197

(56) References cited:
- WO-A1-2010/086452
- US-A1- 2011 295 331
- US-A1- 2012 197 374

## Description

The invention relates to a system for stimulation of a patient's cochlea.

The sense of hearing in human beings involves the use of hair cells in the cochlea that convert or transduce acoustic signals into auditory nerve impulses. Hearing loss, which may be due to many different causes, is generally of two types: conductive and sensorineural. Conductive hearing loss occurs when the normal mechanical pathways for sound to reach the hair cells in the cochlea are impeded. These sound pathways may be impeded, for example, by damage to the auditory ossicles. Conductive hearing loss may often be overcome through the use of conventional hearing aids that amplify sound so that acoustic signals can reach the hair cells within the cochlea. Some types of conductive hearing loss may also be treated by surgical procedures.

Cochlear hearing loss, on the other hand, is caused by the absence or destruction of the hair cells in the cochlea which are needed to transduce acoustic signals into auditory nerve impulses. People who suffer from cochlear hearing loss may be unable to derive significant benefit from conventional hearing aid systems, no matter how loud the acoustic stimulus is. This is because the mechanism for transducing sound energy into auditory nerve impulses has been damaged. Thus, in the absence of a properly functioning cochlea, auditory nerve impulses cannot be generated directly from sounds.

To overcome sensorineural hearing loss, numerous auditory prosthesis systems (e.g., cochlear implant systems) have been developed. Auditory prosthesis systems bypass the hair cells in the cochlea by presenting electrical stimulation directly to the auditory nerve fibers. Direct stimulation of the auditory nerve fibers leads to the perception of sound in the brain and at least partial restoration of hearing function.

To facilitate direct stimulation of the auditory nerve fibers, a lead having an array of electrodes disposed thereon may be implanted in the cochlea of a patient. The electrodes form a number of stimulation channels through which electrical stimulation pulses may be applied directly to auditory nerve fibres within the cochlea. An audio signal may then be presented to the patient by translating the audio signal into a number of electrical stimulation pulses and applying the stimulation pulses directly to the auditory nerve within the cochlea via one or more of the electrodes.

Typically, the audio signal, which usually is captured by a microphone, is divided into a plurality of analysis channels, each containing a signal representative of a distinct frequency portion of the audio signal, wherein the frequency domain signal in each analysis channel may undergo signal processing, such as by applying channel-specific gain to the signals. The processed signals are used for generating certain stimulation parameters according to which the stimulation signals in each stimulation channel are generated. The analysis channels are linked to the stimulation channels via channel mapping. The number of stimulation channels may correspond to the number of analysis channels, or there may be more stimulation channels than analysis channels, or there may be more analysis channels than stimulation channels. Various stimulation strategies are used, such as current steering stimulation (in order to stimulate a stimulation site located in between areas associated with two or more electrodes) and N-of-M stimulation (wherein stimulation current is only applied to N of M total stimulation channels during a particular stimulation frame where N is less than or equal to M).

An example for such a CI system with electrical cochlear stimulation is described in WO 2011/032021 A1.

It is also known that the cochlea may be stimulated by light, see for example, AD Izzo et al., "Laser stimulation of the auditory nerve", Lasers in surgery and medicine 38, 2006; AD Izzo et al., "Selectivity of neural stimulation in the auditory system: a comparison of optic and electric stimuli", Journ. Biomed. Optics 12, 2007 ; and AD Izzo et al., "Laser stimulation of auditory neurons : effect of shorter pulse duration and penetration depth", Biophysical Journal 94, 2008. Optical stimulation results in discrete stimulation of the hair cells without diffusion or overlap.

WO 2009/072123 A2 relates to optical stimulation of the cochlea via a plurality of optical fibers implanted in the cochlea. A similar system is described in US 2006/0161227 A1.

US 6,921,413 B2 relates, in a general manner, to the direct stimulation of neural tissue with optical energy.

WO 2009/079704 A1 mentions that the cochlea may be stimulated either electrically via an implanted electrode array or optically via an optical fiber.

US 2010/0094380 A1 relates to a cochlear implant hearing instrument comprising a lead assembly implanted in the cochlea which comprises in addition to electrode contacts at least one optical contact for providing both electrical and optical stimulation to the cochlea.

US 2010/0114190 A1 mentions that the auditory nerve may be stimulated both by an electrical signal and an optical signal, in particular by using an optical fiber having a metallization layer applied to it, with the optical stimulation signal being applied through the optical fiber and the electrical stimulation signal being applied to the metallization layer.

US 2006/0129210 A1 relates to a cochlear implant comprising a primary wave guide having various output positions along the light guiding axis for optically stimulating auditory neuron sites of the cochlea. The light coupled into the primary wave guide also may be used for being transformed into an electrical stimulation signal to the cochlea.

US 2010/0174330 A2 relates to a CI hearing instrument comprising a stimulation assembly implanted within the cochlea which comprises both electrode contacts for electrical stimulation of the cochlea and light emitting diodes (LEDs) or laser diodes for optical stimulation of the cochlea.

WO 2012/010196 A1 relates to a CI hearing instrument comprising a stimulation assembly including an electrode array to be located in a basal part of the cochlea for electrical stimulation of the auditory nerve in the basal part of the cochlea and a plurality of optical fibers extending beyond the electrode array into the apical part of the cochlea for optical stimulation of the auditory nerve in the apical part of the cochlea.

WO2009/090047 A1 relates to a retinal implant device comprising a photodiode which is powered by a rectified AC (alternating current) supply voltage in order to avoid corrosion which might result from application of an external DC (direct current) supply voltage.

It is known that LEDs can be connected in an anti-parallel (or "back-to-back") configuration to a AC power source, see for example US 2012/0146536 A1 relating to an illumination system for houses or vehicles, US 5,699,283 relating to a fuse assembly, and WO 2011/036489 A1 relating to a power source.

The use of LEDs as a light source requires the application of a DC voltage to drive the LEDs. However, in the case of an LED implanted within the cochlea, direct currents may have severe negative effects on the cochlear tissue in the event of leakage current or a major failure of the device.

It is an object of the invention to provide for a system enabling optical stimulation of the cochlea which enables safe operation and which nevertheless has a relative simple design and/or can be implanted in a relatively Document US-A-2012/197374 discloses the most relevant prior art.

According to the invention, this object is achieved by a cochlear stimulation system as defined in claim 1.

The invention is beneficial, in that, by utilizing LEDs in a "back-to-back" configuration as the light source, with the polarity of the electric stimulation signals supplied to the LEDs being changed periodically, a "balanced" drive signal can be applied to the LEDs, wherein the negative effect of direct current on cochlear tissue can be avoided while nevertheless essentially continuous light emission from the LEDs can be achieved. In particular, by enabling such safe use of LEDs, the need to use fiber optic techniques within the cochlea, which would make the system more complex, is avoided.

Preferably, the time-average of the electrical stimulation signals is zero so that the potential damage of the current applied to the LEDs is minimized.

Typically, the LEDs are spatially arranged in groups with part of the LEDs of each group belonging to the first subgroup (which is active when the electric stimulation signal has the first polarity) and the remaining part of the LEDs of each group belonging to the second subgroup (which is active when the stimulation signal has the second polarity). For example, each group may consist of two LEDs. Preferably, the driver unit and the stimulation assembly are designed for providing the stimulation signal to each group separately, so that stimulation through several frequency channels is enabled.

Preferably, the polarity changes once during each polarity period, with the shape of the stimulation signal during the first polarity being anti-symmetric with regard to the shape of the stimulation signal during the second polarity.

Further, preferred embodiments are defined in the dependent claims.

Hereinafter, examples of the invention will be illustrated by reference to the attached drawings, wherein:
- Fig. 1: is a schematic view of an example of a CI system according to the invention;
- Fig. 2: is a schematic cross-sectional view of a human cochlea with marked stimulation sites;
- Fig. 3: is a block diagram of the signal processing structure of a cochlear implant system according to the invention;
- Fig. 4: is a schematic sectional view of an example of a stimulation assembly according to the invention;
- Fig. 5: shows a modification of the stimulation assembly of Fig. 4;
- Fig. 6: shows a circuit diagram of a LED pair which can be used in the present invention; and
- Fig. 7: is an example of the amplitude vs. time of the stimulation signal applied to a pair of LEDs according to the invention.

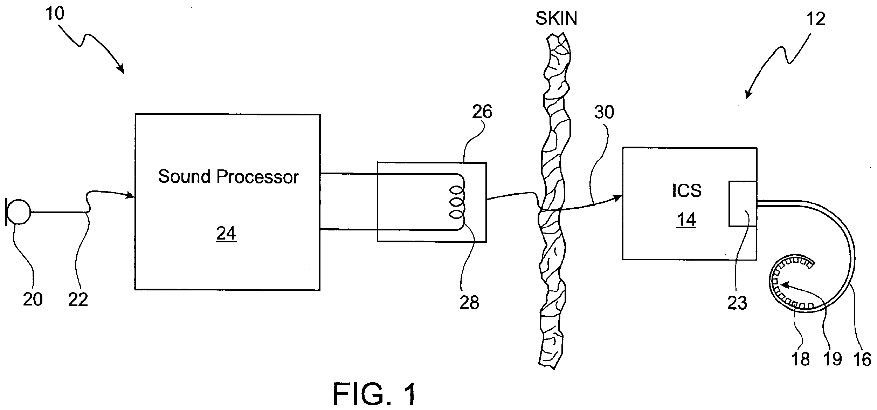

In Fig. 1 an example of a cochlear implant system is shown schematically. The system comprises a sound processing sub-system 10 and a stimulation sub-system 12. The sound processing sub-system 10 serves to detect or sense an audio signal and divide the audio signal into a plurality of analysis channels each containing a signal representative of a distinct frequency portion of the audio signal. A signal level value and a noise level value are determined for each analysis channel by analyzing the respective frequency domain signal, and a noise reduction gain parameter is determined for each analysis channel as a function of the signal level value and the noise level value of the respective analysis channel. Noise reduction is applied to the analysis channel signal according to the noise reduction gain parameters to generate a noise reduced frequency domain signal. Stimulation parameters are generated based on the noise reduced signal and are transmitted to the stimulation sub-system 12.

Stimulation sub-system 12 serves to generate and apply electrical and optical stimulation (also referred to herein as "stimulation current" and/or "stimulation pulses") to stimulation sites at or within the auditory nerve within the cochlear of a patient in accordance with the stimulation parameters received from the sound processing sub-system 10. Electrical and optical stimulation is provided to the patient via a CI stimulation assembly 18 comprising a plurality of stimulation channels, wherein various known stimulation strategies, such as current steering stimulation or N-of-M stimulation, may be utilized.

As used herein, a "current steering stimulation strategy" is one in which weighted stimulation current is applied concurrently to two or more electrodes by an implantable cochlear stimulator in order to stimulate a stimulation site located in between areas associated with the two or more electrodes and thereby create a perception of a pitch in between the frequencies associated with the two or more electrodes, compensate for one or more disabled electrodes, and/or generate a target pitch that is outside a range of pitches associated with an array of electrodes.

As used herein, an "N-of-M stimulation strategy" is one in which stimulation current is only applied to N of M total stimulation channels during a particular stimulation frame, where N is less than M. An N-of-M stimulation strategy may be used to prevent irrelevant information contained within an audio signal from being presented to a CI user, achieve higher stimulation rates, minimize electrode interaction, and/or for any other reason as may serve a particular application.

The stimulation parameters may control various parameters of the electrical and optical stimulation applied to a stimulation site including, but not limited to, frequency, pulse width, amplitude, waveform (e.g., square or sinusoidal), electrode polarity (i.e., anode-cathode assignment), location (i.e., which electrode pair or electrode group receives the stimulation current), burst pattern (e.g., burst on time and burst off time), duty cycle or burst repeat interval, spectral tilt, ramp on time, and ramp off time of the stimulation current that is applied to the stimulation site.

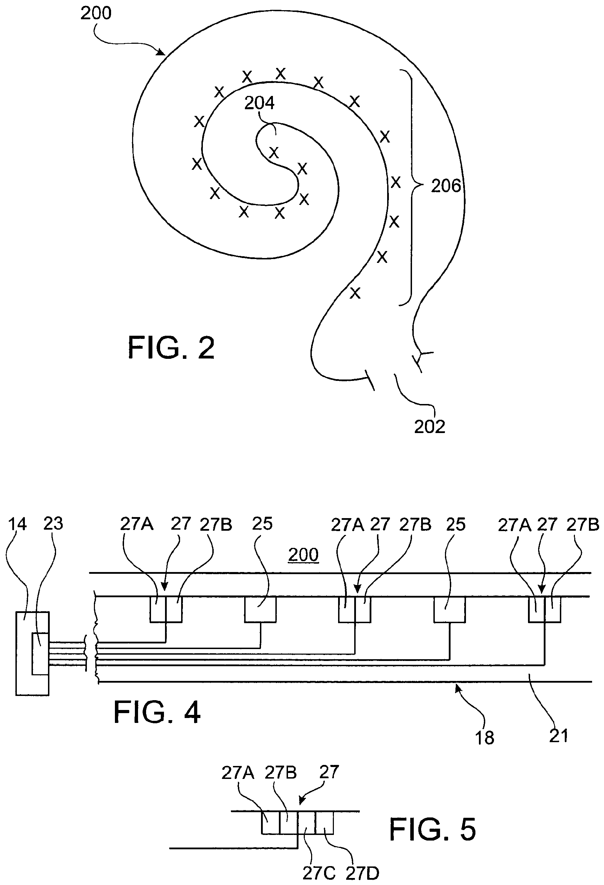

Fig. 2 illustrates a schematic structure of the human cochlea 200. As shown in Fig. 2, the cochlea 200 is in the shape of a spiral beginning at a base 202 and ending at an apex 204. Within the cochlea 200 resides auditory nerve tissue 206, which is denoted by Xs in Fig. 2. The auditory nerve tissue 206 is organized within the cochlea 200 in a tonotopic manner. Low frequencies are encoded at the apex 204 of the cochlea 200 while high frequencies are encoded at the base 202. Hence, each location along the length of the cochlea 200 corresponds to a different perceived pitch. Stimulation subsystem 12 is configured to apply stimulation to different locations within the cochlea 200 (e.g., different locations along the auditory nerve tissue 206) to provide a sensation of hearing.

Returning to Fig. 1, sound processing subsystem 10 and stimulation subsystem 12 may be configured to operate in accordance with one or more control parameters. These control parameters may be configured to specify one or more stimulation parameters, operating parameters, and/or any other parameter as may serve a particular application. Exemplary control parameters include, but are not limited to, most comfortable current levels ("M levels"), threshold current levels ("T levels"), dynamic range parameters, channel acoustic gain parameters, front- and back-end dynamic range parameters, current steering parameters, amplitude values, pulse rate values, pulse width values, polarity values, filter characteristics, and/or any other control parameter as may serve a particular application.

In the example shown in Fig. 1, the stimulation sub-system 12 comprises an implantable cochlear stimulator ("ICS") 14, a lead 16 and the stimulation assembly 18 disposed on the lead 16. The stimulation assembly 18 comprises a plurality of "stimulation contacts" 19 for electrical and optical stimulation of the auditory nerve. The lead 16 may be inserted within a duct of the cochlea in such a manner that the stimulation contacts 19 are in communication with one or more stimulation sites within the cochlea, i.e. the stimulation contacts 19 are adjacent to, in the general vicinity of, in close proximity to, directly next to, or directly on the respective stimulation site; this includes stimulation sites within the auditory nerve or higher auditory pathway.

In the example shown in Fig. 1, the sound processing sub-system 10 is designed as being located external to the patient; however, in alternative examples, at least one of the components of the sub-system 10 may be implantable.

In the example shown in Fig. 1, the sound processing sub-system 10 comprises a microphone 20 which captures audio signals from ambient sound, a microphone link 22, a sound processor 24 which receives audio signals from the microphone 20 via the link 22, and a headpiece 26 having a coil 28 disposed therein. The sound processor 24 is configured to process the captured audio signals in accordance with a selected sound processing strategy to generate appropriate stimulation parameters for controlling the ICS 14 and may include, or be implemented within, a behind-the-ear (BTE) unit or a portable speech processor ("PSP"). In the example of Fig. 1 the sound processor 24 is configured to transcutaneously transmit data (in particular data representative of one or more stimulation parameters) to the ICS 14 via a wireless transcutaneous communication link 30. The headpiece 26 may be affixed to the patient's head and positioned such that the coil 28 is communicatively coupled to the corresponding coil (not shown) included within the ICS 14 in order to establish the link 30. The link 30 may include a bidirectional communication link and/or one or more dedicated unidirectional communication links. According to an alternative embodiment, the sound processor 24 and the ICS 14 may be directly connected by wires.

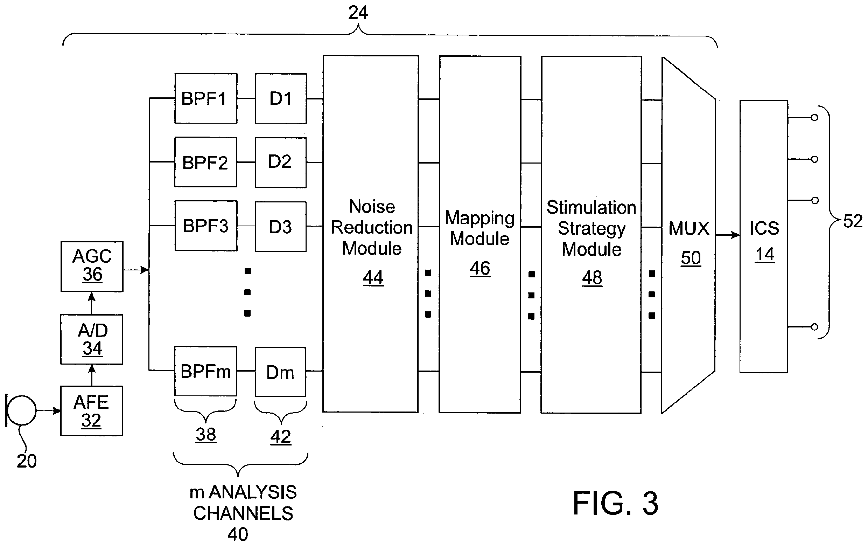

In Fig. 3 a schematic example of a sound processor 24 is shown. The audio signals captured by the microphone 20 are amplified in an audio front end circuitry 32, with the amplified audio signal being converted to a digital signal by an analog-to-digital converter 34. The resulting digital signal is then subjected to automatic gain control using a suitable automatic gain control (AGC) unit 36.

After appropriate automatic gain control, the digital signal is subjected to a plurality of filters 38 (for example, band-pass filters) which are configured to divide the digital signal into *m* analysis channels 40, each containing a signal representative of a distinct frequency portion of the audio signal sensed by the microphone 20. For example, such frequency filtering may be implemented by applying a Discrete Fourier Transform to the audio signal and then divide the resulting frequency bins into the analysis channels 40.

The signals within each analysis channel 40 are input into an envelope detector 42 in order to determine the amount of energy contained within each of the signals within the analysis channels 40 and to estimate the noise within each channel. After envelope detection the signals within the analysis channels 40 are input into a noise reduction module 44, wherein the signals are treated in a manner so as to reduce noise in the signal in order to enhance, for example, the intelligibility of speech by the patient. Some possible examples of the noise reduction module 44 are described e.g. in WO 2011/032021 A1.

The noise reduced signals are supplied to a mapping module 46 which serves to map the signals in the analysis channels 40 to the stimulation channels. For example, signal levels of the noise reduced signals may be mapped to amplitude values used to define the electrical stimulation pulses that are applied to the patient by the ICS 14 via M stimulation channels 52. For example, each of the m stimulation channels 52 may be associated to one of the stimulation contacts 19 or to a group of the stimulation contacts 19.

The sound processor 24 further comprises a stimulation strategy module 48 which serves to generate one or more stimulation parameters based on the noise reduced signals and in accordance with a certain stimulation strategy (which may be selected from a plurality of stimulation strategies). For example, stimulation strategy module 48 may generate stimulation parameters which direct the ICS 14 to generate and concurrently apply weighted stimulation current via a plurality of the stimulation channels 52 in order to effectuate a current steering stimulation strategy. Additionally or alternatively the stimulation strategy module 48 may be configured to generate stimulation parameters which direct the ICS 14 to apply electrical stimulation via only a subset N of the stimulation channels 52 in order to effectuate an N-of-M stimulation strategy.

The sound processor 24 also comprises a multiplexer 50 which serves to serialize the stimulation parameters generated by the stimulation strategy module 48 so that they can be transmitted to the ICS 14 via the communication link 30, e.g. via the coil 28.

In Fig. 4 an example of the stimulation assembly 18 of Fig. 1 is shown, wherein the stimulation contacts 19 are embedded within a carrier element 21 in such a manner that the stimulation contacts 19 are distributed spaced apart from each other in the longitudinal direction of the stimulation assembly 18. Each stimulation contact 19 is connected via wire(s) or other conductive elements, embedded within the carrier element 21 to a driver unit 23 included within the ICS 14.

Some of the stimulation contacts 19 are formed by stimulation electrodes 25 for electrical stimulation of the auditory nerve. The other stimulation contacts 19 are formed by LED groups 27. In the example of Fig. 4, the electrodes 25 and the LED groups 27 are arranged in a spatially interleaved manner, so that both the LED groups 27 and the electrodes 25 are distributed along the longitudinal direction of the stimulation assembly 18. However, alternative arrangements are conceivable; for example, the electrodes 25 may be arranged to be located in a first part of the cochlea, for example the basal part, and the LED groups 27 may be arranged to be located in another part of the cochlea, for example apical part.

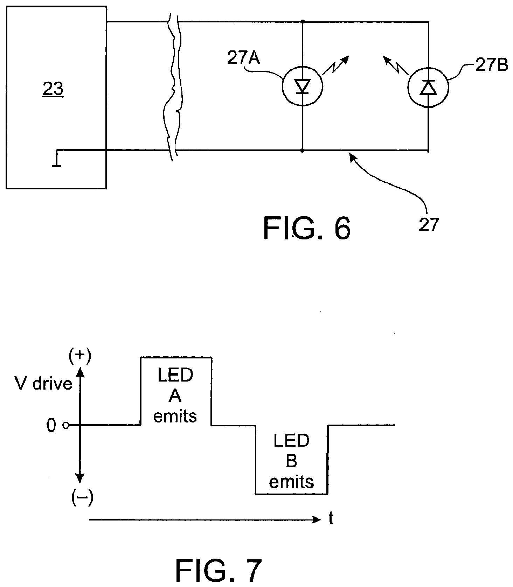

In the example of Fig. 4, each LED group consists of two LEDs 27A, 27B which are connected to the driver unit 23 of the ICS 14 in such a manner that, when the electric stimulation signal applied to the LEDs has a first polarity, one of the LEDs (e.g. the LED 27A) is active (i.e. is forward biased to emit light) while the other one of the LEDs (i.e. the LED 27B) is inactive (i.e. is reverse biased). In other words, the LEDs 27A, 27B are connected in a back-to-back (or anti-parallel) configuration as shown in Fig. 6. Consequently, when the stimulation signal applied to the LEDs 27A, 27B has a second polarity opposite to the first polarity, the formerly inactive one of the LEDs (i.e. the LEDs 27B) now is active, whereas the formally active one (i.e. the LED 27A) now is reverse biased and thus inactive. In other words, the LEDs 27A, 27B of each LED group 27 are connected such that respective of the polarity of the stimulation signal applied to the respective LED group 27 always one of the LEDs is forward biased and the other one is reverse biased.

The electric stimulation signal for each of the LED groups 27 is generated by the driver unit 23 in such a manner that the polarity of the signal is changed periodically. Preferably, the signal is generated in such a manner that the time-average of the electric stimulation signal is zero, so that there is no total DC current applied to the cochlear tissue.

An example of one polarity period of a stimulation signal is shown in Fig. 7, wherein the polarity changes once during each polarity period, wherein the time shape of the signal during the times of the first polarity is anti-symmetric with regard to the time shape of the signal during times of the second polarity. As shown in example of Fig. 7, each polarity period of the signal may comprise a first pulse having the first polarity and a second pulse having the second polarity. As shown in Fig. 7, the pulses may be separated by a time interval wherein the signal amplitude is zero. In the example of Fig. 7, the LED 27A would be active during the first pulse, and the LED 27B would be active during the second pulse. For example, the length of each polarity period may be from 10 µs to 100 ms and the duration of each pulse may from 5 µs to 500µs.

In the example of Fig. 4, each LED group 27 consists of two LEDs and each LED group 27 may be separately provided with the stimulation signal, i.e. different LED groups maybe used in different channels.

However, alternative embodiments are conceivable. For example, each LED group (or some of the LED groups) 27 may consist of more than two LEDs, for example of four LEDs 27A, 27B, 27C and 27D, as shown in Fig. 5. In this case, two of the LEDs (for example the LEDs 27A and 27C) may form a first subgroup by being connected in parallel, while the remaining LEDs 27B, 27D form another subgroup by being connected in parallel with regard to each other, but anti-parallel with regard to the LEDs of the first subgroup.

Preferably, the LEDs of each LED group are arranged for having a substantially similar spatial light emission profile, i.e. the LEDs are of the same type and emit into the same direction.

Preferably, the LEDs of each LED group are located immediately adjacent. Thereby, it is ensured that the light emission profile - and hence the stimulation experienced by the auditory nerve - changes only little when the polarity of the stimulation signal changes.

It is also conceivable that LEDs of different LED groups are connected to form a subgroup in the sense that the same signal is applied to them and they emit light upon the same polarity (in other words, they are connected in parallel).

While in Fig. 4 an example is shown wherein there is both electrical and optical stimulation of the auditory nerve, embodiments are conceivable wherein there is optical stimulation only.

## Claims

1. A system for stimulation of a patient's cochlea, comprising
means (20) for providing an audio signal;
a sound processor (24) for generating auditory nerve stimulation signals from the audio signal,
a stimulation assembly (18) for being implanted within the cochlea and comprising an array of LEDs (27A, 27B, 27C, 27D),
a driver unit (14, 23) for providing the stimulation assembly with electric stimulation signals according to the auditory nerve stimulation signals,
wherein the LEDs are arranged for optically stimulating the auditory nerve in the cochlea according to the electric stimulation signals, wherein the LEDs are connected to the driver unit in such a manner that, when the electric stimulation signal has a first polarity a first subgroup (27A, 27C) of the LEDs is active and a second subgroup (27B, 27D) of the LEDs is inactive, and when the electric stimulation signal has a second polarity opposite to the first polarity, the second subgroup of the LEDs is active and the first subgroup of the LEDs is inactive, and wherein the driver unit is for generating the electric stimulation signals in such a manner that the polarity of the electric stimulation signals is changed periodically.

2. The system of claim 1, wherein the time-average of the electric stimulation signals is zero.

3. The system of one of claims 1 and 2, wherein the LEDs (27A, 27B, 27C, 27D) are spatially arranged in groups (27), wherein one part (27A, 27C) of the LEDs of each group belongs to the first subgroup and the remaining part of the LEDs (27B, 27D) of each group belongs to the second subgroup.

4. The system of claim 3, wherein the LEDs (27A, 27B, 27C, 27D) of each group (27) are located immediately adjacent.

5. The system of claim 4, wherein the LEDs (27A, 27B, 27C, 27D) of each group (27) are arranged for having a substantially similar spatial light emission profile.

6. The system of one of claims 3 to 5, wherein the driver unit (14, 23) and the stimulation assembly (18) are designed for providing the stimulation signal to each group (27) separately.

7. The system of one of claims 3 to 6, wherein each group (27) consists of two LEDs (27A, 27B).

8. The system of one of claims 3 to 7, wherein the stimulation assembly (18) is designed to extend in the longitudinal direction within the cochlea (200), and wherein the LED groups (27) are arranged spaced apart from each other in the longitudinal direction.

9. The system of claim 8, wherein the stimulation assembly (18) comprises a plurality of stimulation electrodes (25) for electrical stimulation of the auditory nerve, and wherein the stimulation electrodes are arranged spaced apart from each other in the longitudinal direction of the stimulation assembly.

10. The system of claim 9, wherein the LED groups (27) and the stimulation electrodes (25) are arranged in a spatially interleaved manner.

11. The system of one of the preceding claims, wherein the driver unit (14, 23) is designed such that during each polarity period the polarity changes once.

12. The system of claim 11, wherein the driver unit (14, 23) is designed such that within each polarity period that the signal shape during the first polarity is anti-symmetric with regard to the signal shape during the second polarity.

13. The system of claim 12, wherein the driver unit (14, 23) is designed such that each polarity period comprises a first pulse having the first polarity and a second pulse having the second polarity.

14. The system of claim 13, wherein the pulses are separated by an interval of zero signal.

15. The system of one of the preceding claims, wherein the length of each polarity period is from 10 µs to 100 ms

## Patentansprüche

1. System zum Stimulieren der Cochlea eines Patienten, mit:
Mitteln (20) zum Bereitstellen eines Audiosignals;
einem Schallprozessor (24) zum Erzeugen von Hörnerv-Stimulationssignalen aus dem Audiosignal;
einer Stimulationsbaugruppe (18) zum Implantieren innerhalb der Cochlea, die ein Array von LEDs (27A, 27B, 27C, 27D) enthält;
einer Treibereinheit (14, 23) zum Versorgen der Stimulationsbaugruppe mit elektrischen Stimulationssignalen gemäß den Hörnerv-Stimulationssignalen;
wobei die LEDs zum optischen Stimulieren des Hörnervs in der Cochlea gemäß den elektrischen Stimulationssignalen angeordnet sind, wobei die LEDs mit der Treibereinheit so verbunden sind, dass, wenn das elektrische Stimulationssignal eine erste Polarität hat, eine erste Untergruppe (27A, 27C) der LEDs aktiv ist und eine zweite Untergruppe (27B, 27D) der LEDs inaktiv ist, und, wenn das elektrische Stimulationssignal eine zweite Polarität entgegengesetzt zur ersten Polarität hat, die zweite Untergruppe der LEDs aktiv ist und die erste Untergruppe der LEDs inaktiv ist, und wobei die Treibereinheit zum Erzeugen der elektrischen Stimulationssignale dergestalt vorgesehen ist, dass die Polarität des elektrischen Stimulationssignals periodisch geändert wird.

2. System gemäß Anspruch 1, wobei das zeitliche Mittel der elektrischen Stimulationssignale null ist.

3. System gemäß Anspruch 1 und 2, wobei die LEDs (27A, 27B, 27C, 27D) räumlich in Gruppen (27) angeordnet sind, wobei ein Teil (27A, 27C) der LEDs jeder Gruppe zu der ersten Untergruppe gehört und der verbleibende Teil der LEDs (27B, 27D) jeder Gruppe zu der zweiten Untergruppe gehört.

4. System gemäß Anspruch 3, wobei die LEDs (27A, 27B, 27C, 27D) einer jeden Gruppe (27) unmittelbar benachbart angeordnet sind.

5. System gemäß Anspruch 4, wobei die LEDs (27A, 27B, 27C, 27D) jeder Gruppe (27) angeordnet sind, um ein im Wesentlichen ähnliches räumliches Lichtemissionsprofil aufzuweisen.

6. System gemäß einem der Ansprüche 3 bis 5, wobei die Treibereinheit (14, 23) und die Stimulationsbaugruppe (18) ausgebildet sind, um das Stimulationssignal jeder Gruppe (27) getrennt bereitzustellen.

7. System gemäß einem der Ansprüche 3 bis 6, wobei jede Gruppe (27) aus zwei LEDs (27A, 27B) besteht.

8. System gemäß einem der Ansprüche 3 bis 7, wobei die Stimulationsbaugruppe (18) ausgebildet ist, um sich in der Längsrichtung innerhalb der Cochlea (200) zu erstrecken, und wobei die LED-Gruppen (27) räumlich getrennt voneinander in der Längsrichtung angeordnet sind.

9. System gemäß Anspruch 8, wobei die Stimulationsbaugruppe (18) eine Mehrzahl von Stimulationselektroden (25) zur elektrischen Stimulation des Hörnervs aufweist, und wobei die Stimulationselektroden räumlich getrennt voneinander in der Längsrichtung der Stimulationsbaugruppe angeordnet sind.

10. System gemäß Anspruch 9, wobei die LED-Gruppen (27) und die Stimulationselektroden (25) in einer räumlich verzahnten Weise angeordnet sind.

11. System gemäß einem der vorhergehenden Ansprüche, wobei die Treibereinheit (14, 23) so ausgebildet ist, dass während jeder Polaritätsperiode die Polarität einmal wechselt.

12. System gemäß Anspruch 11, wobei die Treibereinheit (14, 23) so ausgebildet ist, dass innerhalb jeder Polaritätsperiode die Signalform während der ersten Polarität antisymmetrisch bezüglich der Signalform während der zweiten Polarität ist.

13. System gemäß Anspruch 12, wobei die Treibereinheit (14, 23) so ausgebildet ist, dass jede Polaritätsperiode einen ersten Puls mit der ersten Polarität und einen zweiten Puls mit der zweiten Polarität aufweist.

14. System gemäß Anspruch 13, wobei die Pulse durch ein Intervall mit Null-Signal getrennt sind.

15. System gemäß einem der vorhergehenden Ansprüche, wobei die Länge jeder Polaritätsperiode zwischen 10 µs und 100 ms beträgt.

## Revendications

1. Système de stimulation de la cochlée d'un patient, comprenant :
des moyens (20) pour délivrer un signal audio ;
un processeur de sons (24) pour générer des signaux de stimulation du nerf auditif à partir du signal audio,
un ensemble de stimulation (18) destiné à être implanté dans la cochlée, et comprenant un réseau de LED (27A, 27B, 27C, 27D),
une unité de commande (14, 23) destinée à fournir un ensemble de simulation avec des signaux de stimulation électrique en fonction des signaux de stimulation du nerf auditif,
où les LED sont disposées pour stimuler optiquement le nerf auditif dans la cochlée en fonction des signaux de stimulation électriques, où les LED sont connectées à l'unité de commande de telle sorte que, lorsque le signal de stimulation électrique a une première polarité, un premier sous-groupe (27A , 27C) des LED est actif et un second sous-groupe (27B, 27D) des LED est inactif, et lorsque le signal de stimulation électrique a une seconde polarité opposée à la première polarité, le second sous-groupe de LED est actif et le premier sous-groupe de LED est inactif, et où l'unité de commande est destinée à générer les signaux de stimulation électriques de telle sorte que la polarité des signaux de stimulation électriques est modifiée périodiquement.

2. Système selon la revendication 1, dans lequel la moyenne temporelle des signaux de stimulation électrique est nulle.

3. Système selon l'une des revendications 1 et 2, dans lequel les LED (27A, 27B, 27C, 27D) sont spatialement disposées en groupes (27), où une partie (27A, 27C) des LED de chaque groupe appartient au premier sous-groupe et la partie restante des LED (27B, 27D) de chaque groupe appartient au second sous-groupe.

4. Système selon la revendication 3, dans lequel les LED (27A, 27B, 27C, 27D) de chaque groupe (27) sont disposées de manière adjacente.

5. Système selon la revendication 4, dans lequel les LED (27A, 27B, 27C, 27D) de chaque groupe (27) sont disposées pour avoir un profil spatial d'émission de lumière sensiblement similaire.

6. Système selon l'une des revendications 3 à 5, dans lequel l'unité de commande (14, 23) et l'ensemble de stimulation (18) sont conçus pour délivrer le signal de stimulation à chaque groupe (27) séparément.

7. Système selon l'une des revendications 3 à 6, dans lequel chaque groupe (27) se compose de deux LED (27A, 27B).

8. Système selon l'une des revendications 3 à 7, dans lequel l'ensemble de stimulation (18) est conçu pour s'étendre dans la direction longitudinale à l'intérieur de la cochlée (200), et où les groupes de LED (27) sont disposés espacés les uns des autres dans la direction longitudinale.

9. Système selon la revendication 8, dans lequel l'ensemble de stimulation (18) comprend une pluralité d'électrodes de stimulation (25) pour une stimulation électrique du nerf auditif, et où les électrodes de stimulation sont disposées espacées les unes des autres dans la direction longitudinale de l'ensemble de stimulation.

10. Système selon la revendication 9, dans lequel les groupes de LED (27) et les électrodes de stimulation (25) sont disposés de manière à être entrelacées spatialement.

11. Système selon l'une des revendications précédentes, dans lequel l'unité de commande (14, 23) est conçue de sorte que, au cours de chaque période de polarité, la polarité change une fois.

12. Système selon la revendication 11, dans lequel l'unité de commande (14, 23) est conçue de sorte qu'à l'intérieur de chaque période de polarité, la forme du signal au cours de la première polarité est antisymétrique par rapport à la forme du signal au cours de la seconde polarité.

13. Système selon la revendication 12, dans lequel l'unité de commande (14, 23) est conçue de sorte que chaque périodes de polarité comprend une première impulsion ayant la première polarité et une seconde impulsion ayant la seconde polarité.

14. Système selon la revendication 13, dans lequel les impulsions sont séparées par un intervalle de signal nul.

15. Système selon l'une des revendications précédentes, dans lequel la durée de chaque période de polarité s'étend de 10 µs à 100 ms.
